(19) Europäisches Patentamt
European Patent Office
Office européen des brevets

(11) EP 0 978 509 B1

(12) FASCICULE DE BREVET EUROPEEN

(45) Date de publication et mention de la délivrance du brevet: **31.03.2004 Bulletin 2004/14**

(51) Int Cl.[7]: **C07D 231/40**

(21) Numéro de dépôt: **99401909.9**

(22) Date de dépôt: **27.07.1999**

# (54) Procédé de préparation de carboxamide-oximes

Verfahren zur Herstellung von Carboxamidoximen

Process of preparation of carboxamide oximes

(84) Etats contractants désignés:
**AT BE CH CY DE DK ES FI FR GB GR IE IT LI LU MC NL PT SE**

(30) Priorité: **04.08.1998 FR 9809977**

(43) Date de publication de la demande:
**09.02.2000 Bulletin 2000/06**

(73) Titulaire: **ISOCHEM**
**75194 Paris Cedex 04 (FR)**

(72) Inventeurs:
- **Ferruccio, Laurence**
  **91810 Vert le Grand (FR)**
- **Gibert, Dominique**
  **60340 Villers Sous Saint Leu (FR)**
- **Guyselaine, Vergne**
  **91720 Maisse (FR)**

(74) Mandataire: **Pech, Bernard et al**
**SNPE - Service Propriété Industrielle**
**12, Quai Henri IV**
**75181 Paris Cédex 04 (FR)**

(56) Documents cités:
**EP-A- 0 571 959** **US-A- 4 705 863**

- **CHEMICAL ABSTRACTS, vol. 127, no. 26, 29 décembre 1997 (1997-12-29) Columbus, Ohio, US; abstract no. 358859, SATO T.: "Preparation of N-pyrazolylamidoximes as intermediates for photographic couplers, pharmaceuticals and dyes" XP002099590 & JP 09 278758 A (FUJI PHOTO FILM CO.,LTD.) 28 octobre 1997 (1997-10-28)**
- **CHEMICAL ABSTRACTS, vol. 123, no. 11, 11 septembre 1995 (1995-09-11) Columbus, Ohio, US; abstract no. 143886, MOTOKI M.: "Preparation of imidic acid ester and N-pyrazolylamidoxime compounds as intermediate for color photographic couplers" XP002099591 & JP 07 082252 A (FUJI PHOTO FILM CO LTD) 28 mars 1995 (1995-03-28)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 978 509 B1

## Description

L'invention concerne un nouveau procédé de préparation de carboxamide-oximes. En particulier elle concerne un procédé de préparation de carboxamide-oximes (appelées également amidoximes) N-pyrazolyl substituées et les nouvelles amidines intermédiaires.

Les carboxamide-oximes N-pyrazolyl substituées sont des composés connus utiles comme intermédiaires de synthèse notamment pour la fabrication d'agents de révélation photographique ou de produits pharmaceutiques.

Plusieurs procédés ont été proposés pour les préparer. L'un d'entre eux comporte les quatre étapes suivantes :

> Dans la première étape, on fait réagir une carboxamide non substituée sur l'azote avec un agent de déshydratation, tel que l'oxychlorure de phosphore, afin d'obtenir le nitrile correspondant,
> dans la deuxième étape, le nitrile obtenu est mis à réagir avec un alcool, en milieu acide ou basique, afin d'obtenir un imidate,
> dans la troisième étape, l'imidate est mis à réagir avec un aminopyrazole pour obtenir une amidine monosubstituée sur un atome d'azote,
> dans la quatrième étape, l'amidine monosubstituée est mise à réagir avec l'hydroxylamine pour obtenir l'amidoxime recherchée.

Le schéma réactionnel est le suivant :

$$RCONH_2 + POCl_3 \rightarrow RCN$$

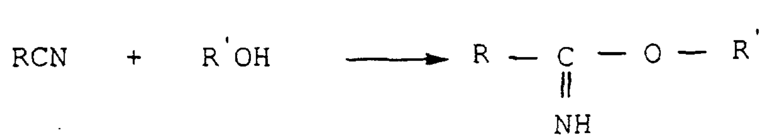

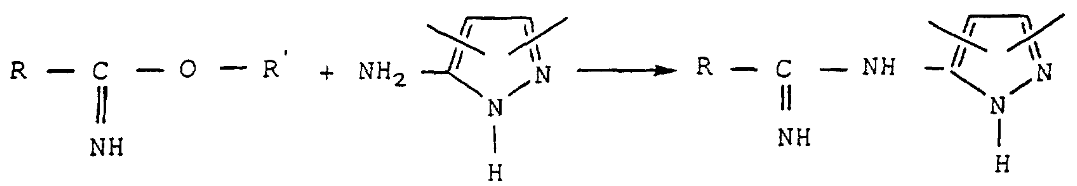

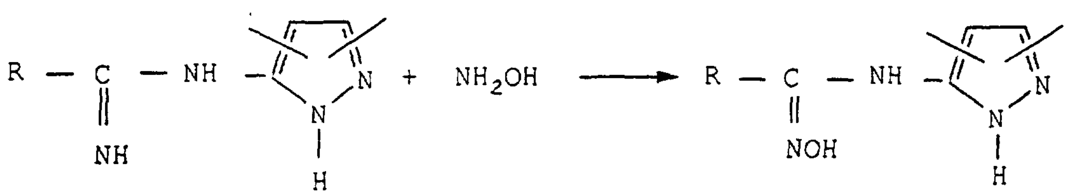

Ce procédé présente des inconvénients. Quatre étapes sont nécessaires à partir de l'amide. Parmi ces étapes, la transformation de l'amide en nitrile est une opération particulièrement délicate. Les nitriles, en particulier les nitriles aromatiques, sont des composés toxiques. Certains sont notamment méthémoglobinisants. Il faut les manipuler avec beaucoup de précaution et ils doivent être éliminés des effluents avant leur rejet. La réaction est effectuée généralement au moyen d'oxychlorure de phosphore, souvent en excès. Le nitrile obtenu est contaminé par des sous-produits, en particulier par des dérivés phosphorés. De nombreux lavages sont alors nécessaires pour éliminer l'excès d'oxychlorure de phosphore et les sous-produits phosphorés. En conséquence une grande quantité d'effluents en résulte. Une certaine quantité de nitrile est entraînée par ces effluents qu'il faut ensuite traiter.

Un autre procédé, décrit dans le brevet US n° 4,705,863, consiste à faire réagir un aminopyrazole avec un ortho-ester pour obtenir un imido-ester. On fait ensuite réagir celui-ci avec l'hydroxylamine pour obtenir la carboxamide-oxime recherchée.

Ce procédé présente également des inconvénients. La préparation des ortho-esters pose des problèmes. Elle s'effectue en deux étapes, soit en passant par un nitrile intermédiaire avec les mêmes désavantages qu'indiqués précédemment, soit par chloration d'un dérivé $RCH_3$ pour obtenir l'intermédiaire trichloré $RCCl_3$ qui est mis en réaction avec un alcool. Mais les dérivés trichlorés possèdent également des propriétés toxiques et irritantes. Ils sont de plus toujours obtenus en mélange avec d'autres dérivés chlorés. La réaction avec l'alcool conduit elle aussi à des mélanges de dérivés alcoylés.

Le schéma réactionnel est le suivant :

$$R - CH_3 \xrightarrow{Cl_2} R - CCl_3$$

$$R - CCl_3 + R'OH \rightarrow R - C - (O - R')_3$$

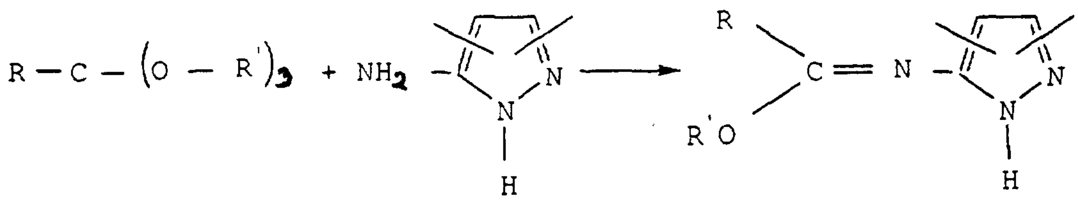

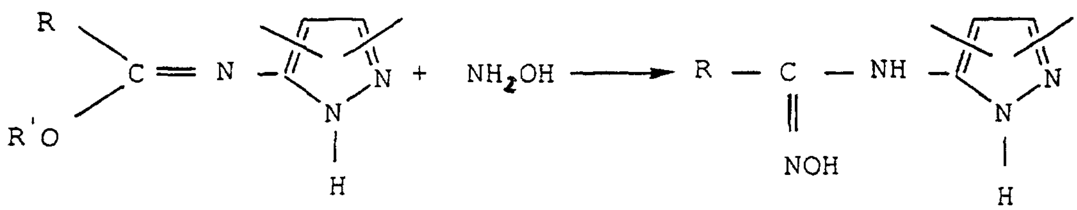

Quatre étapes sont par conséquent également nécessaires pour obtenir les carboxamide-oximes à partir de matières premières courantes.

L'objet de l'invention concerne un procédé de préparation des carboxamide-oximes N-pyrazolyl substituées qui ne présente pas les inconvénients des procédés antérieurs et qui comporte moins d'étapes à effectuer à partir de matières de départ facilement disponibles.

Le procédé selon l'invention consiste à préparer des carboxamide-oximes N-pyrazolyl substituées de formule (I) :

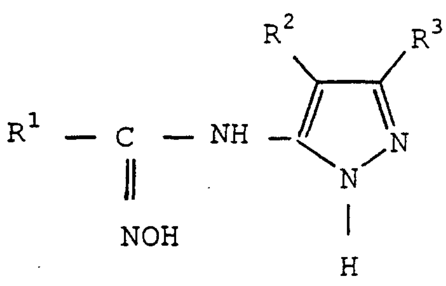

(I)

dans laquelle

$R^1$ représente un groupe aryle ou hétéroaryle comportant un ou plusieurs hétéroatomes choisis parmi l'oxygène, le soufre et l'azote, substitué ou non, le ou les substituants étant choisis dans le groupe constitué par les atomes d'halogène, le groupe nitro, le groupe $CF_3$, les groupes aryles et les groupes hétéroaryles,

$R^2$ représente un atome d'hydrogène ou un atome d'halogène,

$R^3$ représente un atome d'hydrogène ou un groupe alkyle, cycloalkyle, aralkyle ou aryle,

au moyen des étapes suivantes :

1) On fait réagir tout d'abord une amide représentée par la formule $R^1CONHR^4$ (II) dans laquelle $R^1$ a la signification indiquée précédemment et $R^4$ représente un groupe alkyle en $C_1$ à $C_8$, avec un agent chlorurant, éventuellement en présence d'un solvant inerte, pour obtenir la chloroimine correspondante, représentée par la formule (III)

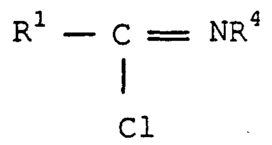

(III)

2) On fait ensuite réagir la chloroimine obtenue avec un aminopyrazole représenté par la formule (IV)

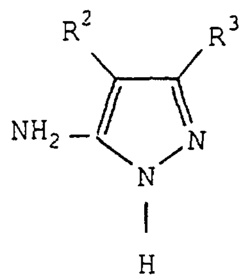

(IV)

dans laquelle $R^2$ et $R^3$ ont les significations indiquées précédemment, éventuellement en présence d'un solvant inerte, pour obtenir une nouvelle amidine dont les deux atomes d'azote portent un substituant, représentée par la formule (V)

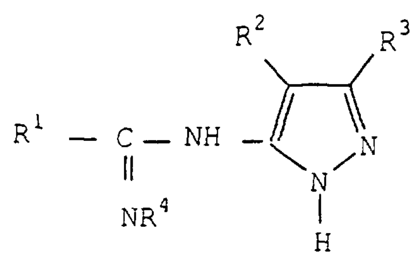

(V)

dans laquelle $R^1$, $R^2$, $R^3$ et $R^4$ ont les significations indiquées précédemment.
3) On fait réagir l'amidine obtenue avec l'hydroxylamine ou un de ses sels, éventuellement en présence d'un solvant inerte.

Le schéma réactionnel est le suivant :

1) 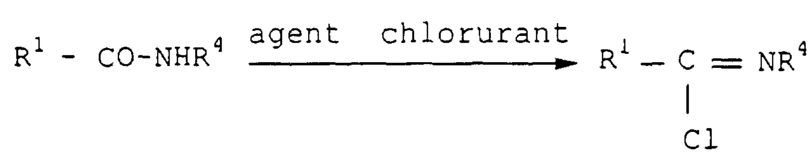

2) 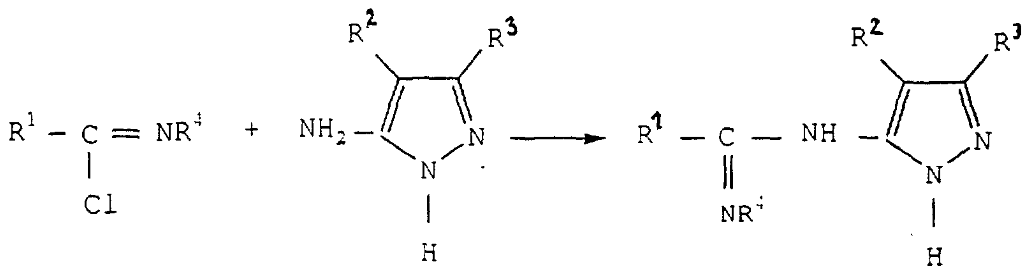

3) 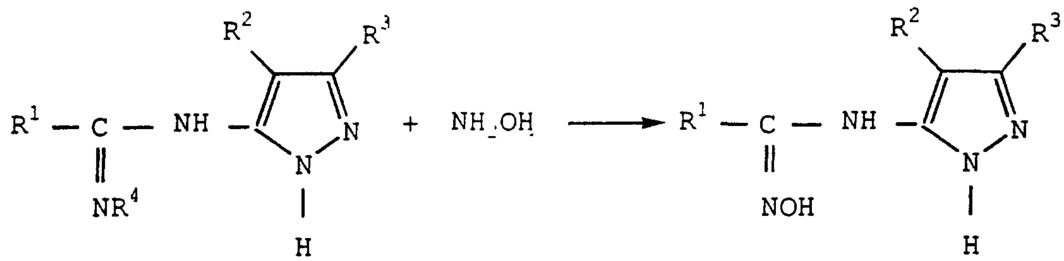

Le nouveau procédé selon l'invention est particulièrement avantageux. Il ne comporte que trois étapes. L'obtention des carboxamide-oximes est par conséquent plus rapide et ce d'autant plus que les deux premières étapes ont une durée plus courte que celle des procédés antérieurs. Dans aucune des étapes, on ne forme un intermédiaire nitrile ou trichloré qui sont des composés très toxiques.

Dans les composés de formule (I), (II), (III) et (V), les substituants de $R^1$ qui représente un groupe aryle ou hétéroaromatique, sont des groupes inertes dans les conditions réactionnelles utilisées, et sont en général choisis dans le groupe constitué par les atomes d'halogène à savoir les atomes de fluor, de chlore, de brome ou d'iode, le groupe $CF_3$, le groupe nitro, les groupes aryles ou hétéroaryles substitués ou non.

Le groupe aryle que représente $R^1$ est généralement un groupe phényle ou naphtyle. De préférence, $R^1$ représente le groupe 4-nitrophényle.

Lorsque $R^1$ représente un groupe hétéroaryle, celui-ci peut comporter un ou plusieurs hétéroatomes tels que l'oxygène, le soufre ou l'azote et est par exemple le furanne, le thiophène, la pyridine ou la pyrimidine.

Le radical $R^2$ porté par le cycle pyrazole dans les composés de formule (I), (IV) et (V) représente un atome d'hydrogène, un atome d'halogène, à savoir un atome de fluor, de chlore, de brome ou d'iode, et de préférence de chlore. $R^2$ représente de préférence un atome d'hydrogène.

Le radical $R^3$, autre radical porté par le cycle pyrazole dans les composés de formule (I), (IV) et (V) peut avoir de nombreuses significations, et notamment peut représenter un atome d'hydrogène, un groupe alkyle primaire, secondaire ou tertiaire, en $C_1$ à $C_{22}$, tel que le groupe méthyle, éthyle, propyle, isopropyle, butyle, isobutyle, tert-butyle, tert-amyle, pentyle, hexyle, heptyle, octyle, décyle, undécyle, tridécyle, octadécyle, un groupe cycloalkyle, aralkyle, aryle.

$R^3$ est de préférence un groupe alkyle en $C_1$ à $C_4$, substitué ou non, plus particulièrement un groupe alkyle tertiaire tel que le groupe tert-butyle.

Le radical $R^4$ contenu dans l'amide de départ de formule (II) et que l'on retrouve dans les composés de formule (III) et (V) est un groupe alkyle en $C_1$ à $C_8$ et de préférence en $C_1$ à $C_4$, tel que le groupe méthyle, éthyle, propyle et butyle. De préférence, $R^4$ représente le groupe méthyle ou éthyle.

Les trois étapes du procédé vont ci-après être décrites de façon plus détaillée. Dans cette description (les exemples non inclus) et dans les revendications correspondantes tous les nombres exprimant des quantités de composés ou des conditions réactionnelles doivent être entendus comme précédés du terme environ.

Pour réaliser l'étape 1), on utilise généralement un agent chlorurant tel que notamment le chlorure de thionyle ($SOCl_2$), le pentachlorure de phosphore ($PCl_5$), l'oxychlorure de phosphore ($POCl_3$), le phosgène ($COCl_2$) ou un de leurs mélanges. De préférence, on utilise le chlorure de thionyle.

L'amide de départ de formule (II) est un composé qui se trouve dans le commerce ou qui peut se préparer selon des méthodes connues telle que par exemple à partir d'un halogénure d'acyle et d'une amine.

L'agent chlorurant est employé en quantité stoechiométrique ou en excès. Pour des raisons économiques, la quantité d'agent chlorurant est de préférence de 1 à 1,25 mole par mole d'amide.

La réaction peut être effectuée sans solvant, l'agent chlorurant servant alors de solvant ou en présence d'un

solvant ou d'un mélange de solvants inertes dans les conditions réactionnelles, choisis parmi les hydrocarbures aromatiques chlorés ou non, tels que le toluène, les xylènes, le monochlorobenzène ou les dichlorobenzènes, les hydrocarbures aliphatiques chlorés ou non, tels que l'éthane ou le dichlorométhane. Le toluène convient bien.

La température de la réaction est généralement comprise entre 25°C et la température de reflux du solvant. Lorsque le toluène est le solvant choisi et l'agent chlorurant est le chlorure de thionyle, elle est en particulier comprise entre 70°C et 110°C.

Des catalyseurs peuvent être ajoutés pour accélérer la réaction, tels que des amides N,N-dialkylées et notamment des formamides dialkylées dont les groupes alkyles ont de 1 à 8 atomes de carbone, comme la N,N-diméthylformamide et plus particulièrement la N,N-dibutylformamide.

La chloruration dure en général entre 2 et 15 heures. La réaction terminée, il n'est pas nécessaire d'isoler la chloroimine qui s'est formée du milieu réactionnel.

Pour réaliser l'étape 2) on fait ensuite réagir l'aminopyrazole de formule (IV) avec la chloroimine obtenue. L'aminopyrazole utilisé est un composé qui se trouve dans le commerce ou que l'on prépare selon des méthodes connues.

La quantité de l'aminopyrazole utilisée est généralement la quantité stoechiométrique ou un excès, notamment, un excès pouvant aller jusqu'à 0,5 mole par mole de chloroimine. Pour des raisons de coût, on préfère utiliser de 1 à 1,25 mole d'aminopyrazole par mole de chloroimine.

La réaction est généralement effectuée en présence d'un solvant ou d'un mélange de solvants inertes dans les conditions réactionnelles, choisis parmi les alcools aliphatiques en $C_1$ à $C_8$, les hydrocarbures aromatiques chlorés ou non, tels que le toluène, les xylènes, le monochlorobenzène ou les dichlorobenzènes, les hydrocarbures aliphatiques chlorés ou non, les éthers tels que le tétrahydrofuranne, les esters tels que l'acétate d'éthyle ou d'isopropyle. De préférence, on utilise les alcools et en particulier le méthanol et/ou l'isopropanol.

La réaction est exothermique et le milieu réactionnel est généralement maintenu à une température allant de -10°C à +30°C, de préférence entre 0°C et 15°C afin d'éviter les réactions secondaires.

On peut neutraliser l'acide chlorhydrique qui se forme, en ajoutant une base, telle que la triéthylamine, l'acétate de sodium ou la pyridine, mais ce n'est pas une variante préférée.

Le procédé selon l'invention permet d'obtenir les amidines de formule (V) qui sont des composés nouveaux. On a trouvé, que de façon inattendue, la fonction amine substituée sur le cycle pyrazole est la fonction réactive qui se fixe sur le carbone de la chloroimine. On pouvait en effet s'attendre à ce que l'un des azotes du cycle soit plus réactif ou réagisse concurrentiellement comme dans d'autres réactions de l'art antérieur mettant en oeuvre ces mêmes pyrazoles, et qu'un mélange d'amidines soit obtenu. Les amidines de formule (V) se forment généralement au fur et à mesure de la mise en contact des deux réactifs. La réaction dure en général de 2 à 10 heures.

Lorsque la réaction est terminée, il n'est pas non plus nécessaire d'isoler préalablement l'amidine obtenue, la troisième étape du procédé pouvant être réalisée dans les mêmes solvants qu'indiqués précédemment. On ajoute alors, de préférence, directement l'hydroxylamine ou l'un de ses sels, tels que par exemple un chlorhydrate ou un sulfate dans le milieu réactionnel. Lorsqu'on utilise le chlorhydrate d'hydroxylamine, on opère, de préférence, en présence d'un solvant qui permet sa solubilisation tel qu'un alcool léger et en particulier le méthanol. De préférence, on ajoute également une base qui permet de libérer l'hydroxylamine de son sel afin d'obtenir une réaction plus complète. Des bases telles que des amines tertiaires peuvent être utilisées, par exemple la triéthylamine, la pyridine. De préférence, on utilise l'acétate de sodium.

L'hydroxylamine ou son sel est mise à réagir avec l'amidine, en quantité stoechiométrique ou avec un excès, notamment un excès de 0,5 à 2 moles par mole d'amidine. En raison de la toxicité de l'hydroxylamine et pour des raisons d'économie, on préfère utiliser une quantité de 1,5 à 2 moles d'hydroxylamine par mole d'amidine.

La température de la réaction est généralement comprise entre 0°C et 60°C et de préférence entre 35°C et 45°C. La durée de la réaction est en général de 2 à 10 heures.

De façon surprenante l'amidoxime souhaitée, c'est à dire celle contenant le radical pyrazolyle, se forme avec un excellent rendement. L'amine de formule $R^4NH_2$ se forme en même temps. Étant donné que les deux groupes azotés de la molécule d'amidine de formule (V) sont tous les deux substitués et qu'ils peuvent être tous les deux des groupes partants, il était à craindre que la réaction ne s'effectue avec perte de la partie aminopyrazole ou qu'un mélange de plusieurs amidoximes soit obtenu. Tel n'est pas le cas.

L'amidoxime formée peut être récupérée selon les méthodes classiques, telles que par exemple par élimination des solvants, filtration, lavage avec de l'eau et séchage.

Les amidoximes sont obtenues selon le procédé de l'invention, avec une bonne pureté et un excellent rendement, le rendement de chacune des étapes étant supérieur à 90%. En particulier, lorsque le groupe $R^1$ de l'amide de départ est le radical 4-nitrophényle et l'aminopyrazole est le 3-tert-butyl-5-aminopyrazole, l'amidoxime est obtenue avec des rendements de 75 à 90% et sa pureté est souvent supérieure à 97%. Les différentes étapes du procédé sont chacune plus rapides que celles utilisées dans l'art antérieur et le nombre d'étapes est réduit. Le procédé peut être réalisé en «one pot» sans isoler les intermédiaires. Ces intermédiaires ne sont pas toxiques. Le procédé selon l'invention est par conséquent plus avantageux que les procédés antérieurs.

Les carboxamide-oximes formées sont des composés connus qui peuvent être transformés pour former des composés utiles notamment comme coupleurs pour photographie, colorants sensibles ou produits pharmaceutiques.

L'invention concerne également les amidines de formule (V')

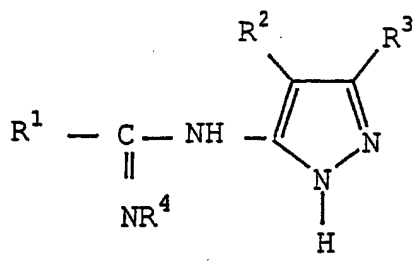

(V')

dans laquelle

$R^1$ représente un groupe aryle ou hétéroaryle comportant un ou plusieurs hétéroatomes choisis parmi l'oxygène, le soufre et l'azote, substitué ou non, le ou les substituants étant choisis dans le groupe constitué par les atomes d'halogène, le groupe nitro, le groupe $CF_3$, les groupes aryles et les groupes hétéroaryles,

$R^2$ représente un atome d'hydrogène ou un atome d'halogène,

$R^3$ représente un atome d'hydrogène ou un groupe alkyle, cycloalkyle, aralkyle ou aryle et

$R^4$ représente un groupe alkyle en $C_1$ à $C_8$,

étant entendu que le groupe aryle représente un groupe phényle ou naphtyle et le groupe hétéroaryle représente le furane, le thiophène, la pyridine ou la pyrimidine.

Les exemples qui suivent illustrent l'invention sans toutefois la limiter.

Dans les exemples 1 à 3, on prépare la N-(3-tertiobutyl-5-pyrazolyl)-4-nitrobenzamide-oxime de formule

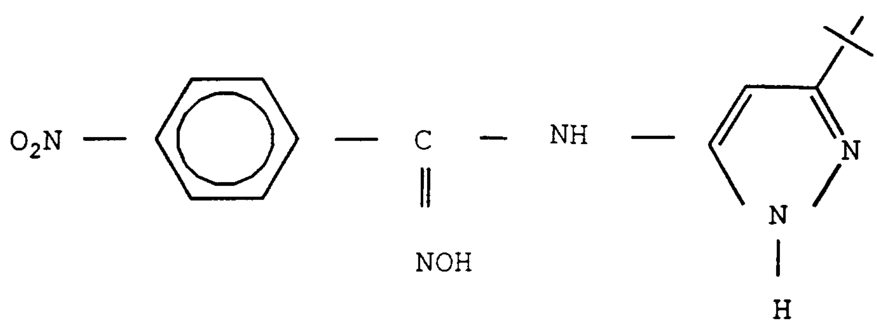

à partir de différentes 4-nitrobenzamides.

Exemple 1 : Préparation à partir de la N-méthyl-4-nitrobenzamide.

Dans un tricol de 100 ml muni d'un agitateur, on introduit 9 g de N-méthyl-4-nitrobenzamide et 40 g de chlorure de thionyle. On chauffe progressivement le mélange jusqu'à la température de reflux du chlorure de thionyle. Le milieu initialement hétérogène devient homogène. Un dégagement gazeux se produit à partir de 65°C et se maintient jusqu'à l'homogénéisation totale du milieu. Il cesse environ 1 heure après le début du chauffage.

On maintient le mélange réactionnel, pendant 1 heure supplémentaire, au reflux du chlorure de thionyle.

On élimine l'excès de chlorure de thionyle par distillation sous pression atmosphérique ou sous pression réduite (50 mm Hg), jusqu'à l'obtention d'une température de masse de 85°-95°C.

On ajoute alors 10 g de toluène dans le milieu réactionnel puis on parachève la distillation de l'excès de chlorure de thionyle, en distillant la plus grande partie du toluène. On ajoute à nouveau 10 g de toluène puis on refroidit le milieu réactionnel vers + 3°C. Le dérivé chloroimine précipite vers 45°C.

On introduit une solution de 6,9 g de 3-tertiobutyl-5-aminopyrazole dans 14 g d'isopropanol, en 1 heure, tout en maintenant la température entre + 5°C et + 15°C.

Le milieu s'épaissit considérablement et devient orangé. On ajoute alors 20 ml d'alcool isopropylique afin d'améliorer l'agitabilité du milieu.

On continue à agiter le mélange à 20°-30°C, pendant 2 heures.

Par un suivi en chromatographie couche mince (CCM), on observe la disparition de l'aminopyrazole et l'apparition de l'amidine.

On ajoute ensuite 20 ml de méthanol puis 7,2 g de chlorhydrate d'hydroxylamine. Le méthanol sert à fluidifier le milieu et favorise la solubilisation du chlorhydrate d'hydroxylamine. On chauffe le milieu réactionnel à 40°-50°C puis on ajoute 3,9 g d'acétate de sodium. Le milieu épaissit considérablement puis se délite en devenant jaune citron.

Le chauffage du milieu réactionnel est maintenu pendant 4 heures. On suit la disparition de l'amidine par CCM.

Lorsque toute l'amidine a disparu, on chasse le mélange de solvants par distillation sous pression réduite (50 mm Hg) jusqu'à l'obtention d'un milieu pâteux jaune citron. On introduit ensuite lentement 50 ml d'eau permutée chauffée à 50°C, sur le concentrat maintenu à 40°- 45°C.

L'amidoxime attendue précipite au cours de l'introduction d'eau. On continue à agiter le mélange, pendant 1 heure, à 40°-45°C.

On recueille le précipité par filtration, puis on le rince avec environ 100 ml d'eau à 50°C puis on sèche le solide jaune citron en étuve.

On obtient alors 12,6 g de l'amidoxime dont la pureté déterminée par CCM est de 95% et déterminée par RMN$^1$H de 96%. En tenant compte de ces déterminations, le rendement obtenu est de 80% en amidoxime pure par rapport à l'amide de départ.

<u>Exemple 2</u> : Préparation à partir de la N-éthyl-4-nitrobenzamide.

Dans un réacteur de 250 l en émail inerté à l'azote, on introduit sous atmosphère d'azote, 45 kg de N-éthyl-4-nitrobenzamide humide (164,1 mol), 65 kg de toluène et 590 g (3,45 mol) de N,N-dibutylformamide. On chauffe le mélange à 85°C puis on introduit 37,2 kg (311 mol) de chlorure de thionyle en 1 heure 45 minutes, tout en maintenant le mélange au voisinage de cette température. Le mélange devient progressivement homogène. De l'acide chlorhydrique et du dioxyde de soufre se dégagent. On continue à agiter le mélange pendant 2 heures à 85°C puis on le refroidit. On élimine l'excès de chlorure de thionyle par distillation du toluène. La distillation terminée, il reste dans le réacteur le dérivé chloroimine et du toluène à une concentration de 50/50 en poids.

On refroidit le milieu réactionnel à 5°C et on ajoute dans le réacteur, 80 kg (172,5 mol) d'une solution à 30% de 3-tertiobutyl-5-aminopyrazole dans l'isopropanol. La réaction est exothermique et dure 5 heures, le milieu s'épaissit et devient orangé. On continue l'agitation pendant 1 heure 30 minutes à une température de 3°C.

On réchauffe le milieu réactionnel à 20°C puis on additionne 55 kg de méthanol. On ajoute ensuite 24 kg (345 mol) de chlorhydrate d'hydroxylamine et on chauffe le milieu réactionnel à 45°C. On ajoute 17 kg (207 mol) d'acétate de sodium et on maintient le chauffage pendant 6 heures. On élimine la plus grande partie des solvants par distillation sous pression réduite. Le milieu réactionnel de couleur jaune devient visqueux. On ajoute 175 kg d'eau distillée à 65°C. L'amidoxime précipite progressivement. Le milieu réactionnel est de couleur orange / ocre. On continue à l'agiter à 45°C, pendant 1 heure. Après filtration et rinçages avec de l'eau puis séchage, on obtient 38,3 kg de l'amidoxime attendue d'une pureté de 98% déterminée par CCM soit un rendement de 77% par rapport à la benzamide de départ.

<u>Exemple 3</u> : Préparation à partir de la N-propyl-4-nitrobenzamide

On opère comme à l'exemple 2, mais en utilisant 20,8 g de N-propyl-4-nitrobenzamide, les autres constituants dans les mêmes proportions molaires, et un réacteur et des conditions opératoires adaptés à ces quantités.

On recueille 24,8 g (rendement 82%) de l'amidoxime attendue d'une pureté de 97% (en RMN$^1$H).

On répète l'expérience, en isolant l'amidine intermédiaire (N-propyl,N'-(3-tertiobutyl-5-pyrazolyl)-4-nitrobenzamidine) sous forme de son chlorhydrate qui présente les caractéristiques suivantes :
spectre RMN$^1$H (DMSO, 200 MHz), $\delta$ (ppm) :
1 (3H,t), 1,35 (9H,s), 1,7 (2H,m), 3,35 (2H,m) 6,15 (1H,s), 7,95 (H de NH,s), 8,02 (2H,d), 8,47 (2H,d), 11,22 (1H, s large), 12,95 (1H, s large).

**Revendications**

1. Procédé de préparation de carboxamide-oximes représentés par la formule générale

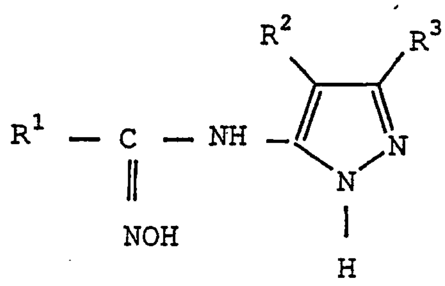

(I)

dans laquelle

$R^1$ représente un groupe aryle ou hétéroaryle comportant un ou plusieurs hétéroatomes choisis parmi l'oxygène, le soufre et l'azote, substitué ou non, le ou les substituants étant choisis dans le groupe constitué par les atomes d'halogène, le groupe nitro, le groupe $CF_3$, les groupes aryles et les groupes hétéroaryles,

$R^2$ représente un atome d'hydrogène ou un atome d'halogène,

$R^3$ représente un atome d'hydrogène ou un groupe alkyle, cycloalkyle, aralkyle ou aryle,

**caractérisé par** les étapes suivantes :

1) On fait réagir tout d'abord une amide représentée par la formule $R^1CONHR^4$ (II) dans laquelle $R^1$ a la signification indiquée précédemment et $R^4$ représente un groupe alkyle en $C_1$ à $C_8$, avec un agent chlorurant, éventuellement en présence d'un solvant inerte, pour obtenir la chloroimine correspondante, représentée par la formule (III)

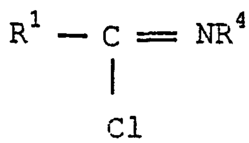

(III)

2) On fait ensuite réagir la chloroimine obtenue avec un aminopyrazole représenté par la formule (IV)

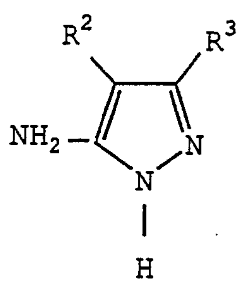

(IV)

dans laquelle $R^2$ et $R^3$ ont les significations indiquées précédemment, éventuellement en présence d'un solvant inerte, pour obtenir une amidine représentée par la formule (V)

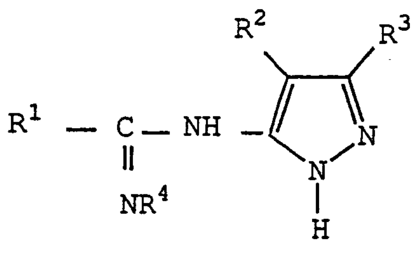

(V)

dans laquelle $R^1$, $R^2$, $R^3$ et $R^4$ ont les significations indiquées précédemment,

3) On fait réagir l'amidine obtenue avec l'hydroxylamine ou un de ses sels, éventuellement en présence d'un solvant inerte.

**2.** Procédé selon la revendication 1, **caractérisé en ce que** le ou les substituants de $R^1$ sont choisis dans le groupe constitué par les atomes d'halogène, le groupe nitro et le groupe $CF_3$.

**3.** Procédé selon la revendication 1 ou 2, **caractérisé en ce que** $R^1$ représente un groupe aryle substitué ou non.

**4.** Procédé selon la revendication 1, 2 ou 3, **caractérisé en ce que** $R^3$ représente un atome d'hydrogène ou un groupe alkyle en $C_1$ à $C_4$.

**5.** Procédé selon la revendication 1, **caractérisé en ce que** $R^1$ représente le groupe 4-nitrophényle, $R^2$ représente un atome d'hydrogène et $R^3$ représente le groupe tert-butyle.

**6.** Procédé selon l'une quelconque des revendications précédentes, **caractérisé en ce que** l'étape 1) est réalisée avec une quantité stoechiométrique ou un excès d'un agent chlorurant choisi dans le groupe constitué par le chlorure de thionyle, le pentachlorure de phosphore, l'oxychlorure de phosphore, le phosgène et un de leurs mélanges, à une température comprise entre environ 25°C et la température de reflux du solvant.

**7.** Procédé selon la revendication 6, **caractérisé en ce que** l'agent chlorurant est le chlorure de thionyle.

**8.** Procédé selon la revendication 7, **caractérisé en ce que** la réaction est effectué en présence d'un catalyseur choisi parmi les amides N,N-dialkylées.

**9.** Procédé selon l'une quelconque des revendications précédentes, **caractérisé en ce que** le solvant de l'étape 1) est choisi dans le groupe constitué par l'agent chlorurant, les hydrocarbures aromatiques chlorés ou non et les hydrocarbures aliphatiques chlorés ou non.

**10.** Procédé selon l'une quelconque des revendications précédentes, **caractérisé en ce que** dans l'étape 2), l'aminopyrazole est mis à réagir avec la chloroimine, en quantité stoechiométrique ou en excès, à une température allant de -10°C à +30°C.

**11.** Procédé selon l'une quelconque des revendications précédentes, **caractérisé en ce que** l'étape 2) est réalisée en présence d'un solvant choisi dans le groupe constitué par les alcools aliphatiques en $C_1$ à $C_8$ , les hydrocarbures aromatiques chlorés ou non, les hydrocarbures aliphatiques chlorés ou non, les éthers et les esters.

**12.** Procédé selon la revendication 11, **caractérisé en ce que** la réaction est effectuée en présence d'un alcool.

**13.** Procédé selon l'une quelconque des revendications précédentes, **caractérisé en ce que** dans l'étape 3), on fait réagir l'hydroxylamine ou un de ses sels, en quantité stoechiométrique ou en excès, à une température allant de 0°C à 60°C.

**14.** Procédé selon l'une quelconque des revendications précédentes, **caractérisé en ce que** dans l'étape 3), on fait réagir le chlorhydrate d'hydroxylamine, en présence du méthanol et d'une base.

**15.** Procédé selon l'une quelconque des revendications précédentes, **caractérisé en ce que** la chloroimine et l'amidine respectivement obtenues dans les étapes 1) et 2) ne sont pas isolées.

**16.** Procédé de préparation des Amidines représentées par la formule générale (V)

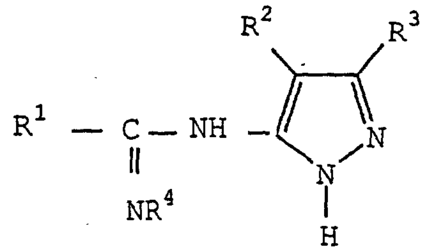

(V)

dans laquelle

$R^1$ représente un groupe aryle ou hétéroaryle comportant un ou plusieurs hétéroatomes cnutsis parmi l'oxygène, le soufre et l'azote, substitué ou non, le ou les substituants étant choisis dans le groupe constitué par les atomes d'halogène, le groupe nitro, le groupe $CF_3$, les groupes aryles et les groupes hétéroaryles,

$R^2$ représente un atome d'hydrogène ou un atome d'halogène,

$R^3$ représente un atome d'hydrogène ou un groupe alkyle, cycloalkyle, aralkyle ou aryle et

$R^4$ représente un groupe alkyle en $C_1$ à $C_8$,

**caractérisé en ce que** les deux premières étapes de synthèse de la revendication 1 sont effectuées.

**17.** Amidines représentées par la formule générale

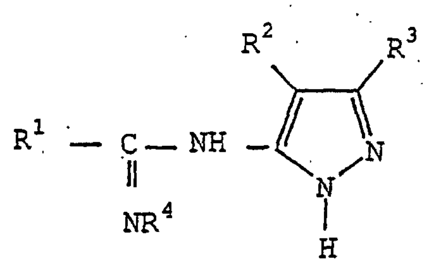

(V)

dans laquelle

$R^1$ représente un groupe aryle ou hétéroaryle, substitué ou non, le ou les substituants étant choisis dans le groupe constitué par les atomes d'halogène, le groupe nitro, le groupe $CF_3$, les groupes aryles et les groupes hétéroaryles,

$R^2$ représente un atome d'hydrogène ou un atome d'halogène,

$R^3$ représente un atome d'hydrogène ou un groupe alkyle, cycloalkle, aralkyle ou aryle et

$R^4$ représente un groupe alkyle en $C_1$ à $C_8$

étant entendu que le groupe aryle représente un groupe phényle ou naphtyle et le groupe hétéroaryle représente le furane, le thiophène, la pyridine ou la pyrimidine.

**18.** Amidines selon la revendication 17, **caractérisé en ce que** le ou les substituants de $R^1$ sont choisis dans le groupe constitué par les atomes d'halogène, le groupe nitro et le groupe $CF_3$.

**19.** Amidines selon la revendication 17 ou 18, **caractérisé en ce que** $R^1$ représente un groupe aryle substitué ou non.

**20.** Amidines selon la revendication 17, 18 ou 19 **caractérisé en ce que** $R^3$ représente un atome d'hydrogène ou un groupe alkyle en $C_1$ à $C_4$.

**21.** Amidines selon la revendication 17, **caractérisé en ce que** $R^1$ représente le groupe 4-nitrophényle, $R^2$ représente un atome d'hydrogène et $R^3$ représente le groupe tert-butyle.

**Patentansprüche**

**1.** Verfahren zur Herstellung von Carboxamidoximen der allgemeinen Formel:

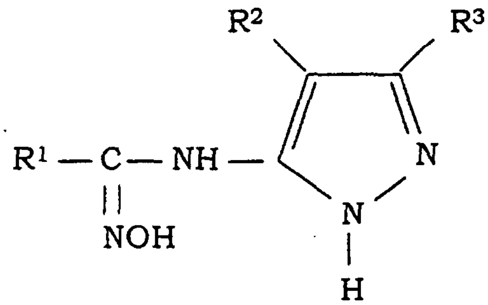

(I),

worin bedeuten:

$R^1$ eine unsubstituierte oder substituierte Arylgruppe oder eine unsubstituierte oder substituierte Heteroarylgruppe, die ein oder mehrere Heteroatome enthält, die unter Sauerstoff, Schwefel und Stickstoff ausgewählt sind, wobei der Substituent oder die Substituenten unter den Halogenatomen, der Nitrogruppe, der Gruppe $CF_3$, Arylgruppen und Heteroarylgruppen ausgewählt sind,
$R^2$ ein Wasserstoffatom oder ein Halogenatom,
$R^3$ ein Wasserstoffatom oder eine Alkylgruppe, Cycloalkylgruppe, Aralkylgruppe oder Arylgruppe,

**gekennzeichnet durch** die folgenden Schritte:

1) zunächst wird ein Amid der Formel $R^1CONHR^4$ (II), worin $R^1$ die oben angegebene Bedeutung aufweist und $R^4$ eine $C_{1-8}$-Alkylgruppe ist, gegebenenfalls in Gegenwart eines inerten Lösungsmittels zur Herstellung des entsprechenden Chlorimins der Formel (III) mit einem Chlorierungsmittel umgesetzt:

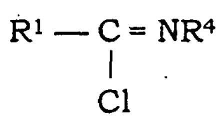

(III),

2) dann wird das erhaltene Chlorimin gegebenenfalls in Gegenwart eines inerten Lösungsmittels mit einem Aminopyrazol der Formel (IV) umgesetzt:

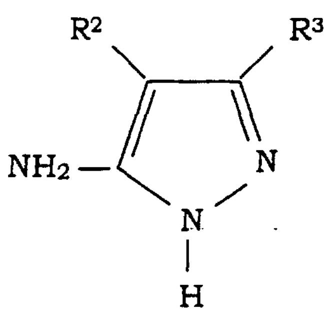

(IV),

worin $R^2$ und $R^3$ die oben angegebenen Bedeutungen aufweisen, um ein Amidin der Formel (V) herzustellen:

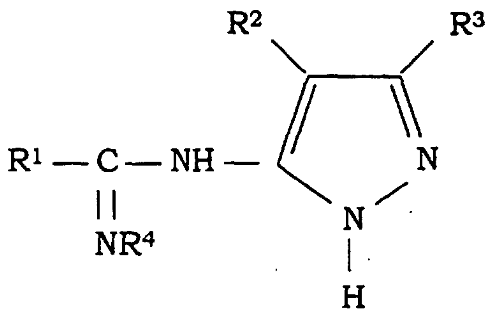

(V),

worin die Gruppen $R^1$, $R^2$, $R^3$ und $R^4$ die oben angegebenen Bedeutungen aufweisen, und

3) das erhaltene Amidin wird gegebenenfalls in Gegenwart eines inerten Lösungsmittels mit Hydroxylamin oder einem seiner Salze umgesetzt.

2. Verfahren nach Anspruch 1, **dadurch gekennzeichnet, dass** der Substituent oder die Substituenten der Gruppe $R^1$ unter den Halogenatomen, der Nitrogruppe und der Gruppe $CF_3$ ausgewählt sind.

3. Verfahren nach Anspruch 1 oder 2, **dadurch gekennzeichnet, dass** $R^1$ eine substituierte oder unsubstituierte Arylgruppe bedeutet.

4. Verfahren nach Anspruch 1, 2 oder 3, **dadurch gekennzeichnet, dass** die Gruppe $R^3$ ein Wasserstoffatom oder eine $C_{1-4}$-Alkylgruppe bedeutet.

5. Verfahren nach Anspruch 1, **dadurch gekennzeichnet, dass** $R^1$ 4-Nitrophenyl, $R^2$ ein Wasserstoffatom und $R^3$ *t*-Butyl bedeutet.

6. Verfahren nach einem der vorhergehenden Ansprüche, **dadurch gekennzeichnet, dass** der Schritt 1) mit einer stöchiometrischen Menge eines Chlorierungsmittels oder mit einem im Überschuss vorliegenden Chlorierungsmittel, welches unter Thionylchlorid, Phosphorpentachlorid, Phosphoroxidtrichlorid, Phosgen und deren Gemischen ausgewählt ist, bei einer Temperatur im Bereich von etwa 25 °C bis zur Rückflusstemperatur des Lösungsmittels durchgeführt wird.

7. Verfahren nach Anspruch 6, **dadurch gekennzeichnet, dass** das Chlorierungsmittel das Thionylchlorid ist.

8. Verfahren nach Anspruch 7, **dadurch gekennzeichnet, dass** die Umsetzung in Gegenwart eines Katalysators durchgeführt wird, der unter den N,N-Dialkylamiden ausgewählt ist.

9. Verfahren nach einem der vorhergehenden Ansprüche, **dadurch gekennzeichnet, dass** das in Schritt 1) verwendete Lösungsmittel unter dem Chlorierungsmittel, chlorierten oder nicht chlorierten aromatischen Kohlenwasserstoffen und chlorierten oder nicht chlorierten aliphatischen Kohlenwasserstoffen ausgewählt ist.

10. Verfahren nach einem der vorhergehenden Ansprüche, **dadurch gekennzeichnet, dass** in Schritt 2) das Aminopyrazol in stöchiometrischer Menge oder im Überschuss mit dem Chlorimin bei einer Temperatur von -10 bis +30 °C umgesetzt wird.

11. Verfahren nach einem der vorhergehenden Ansprüche, **dadurch gekennzeichnet, dass** Schritt 2) in Gegenwart eines Lösungsmittels durchgeführt wird, das unter den aliphatischen $C_{1-8}$-Alkoholen, chlorierten oder nicht chlorierten aromatischen Kohlenwasserstoffen, chlorierten oder nicht chlorierten aliphatischen Kohlenwasserstoffen, Ethern und Estern ausgewählt ist.

12. Verfahren nach Anspruch 11, **dadurch gekennzeichnet, dass** die Umsetzung in Gegenwart eines Alkohols erfolgt.

13. Verfahren nach einem der vorhergehenden Ansprüche, **dadurch gekennzeichnet, dass** in Schritt 3) das Hydroxylamin in stöchiometrischer Menge oder im Überschuss bei einer Temperatur von 0 bis 60 °C umgesetzt wird.

**14.** Verfahren nach einem der vorhergehenden Ansprüche, **dadurch gekennzeichnet, dass** in Schritt 3) das Hydroxylamin-Hydrochlorid in Gegenwart von Methanol und einer Base umgesetzt wird.

**15.** Verfahren nach einem der vorhergehenden Ansprüche, **dadurch gekennzeichnet, dass** das Chlorimin bzw. das Amidin, die in den Schritten 1) und 2) erhalten werden, nicht abgetrennt werden.

**16.** Verfahren zur Herstellung von Amidinen der allgemeinen Formel (V):

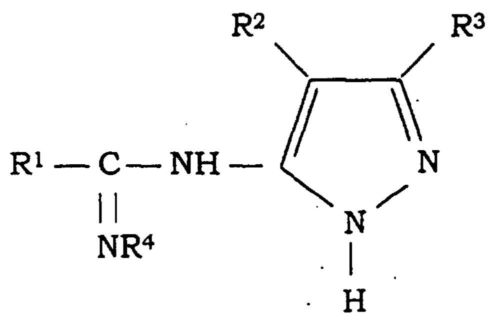

(V),

worin bedeuten:

$R^1$ eine unsubstituierte oder substituierte Arylgruppe oder eine unsubstituierte oder substituierte Heteroarylgruppe, die ein oder mehrere Heteroatome enthält, die unter Sauerstoff, Schwefel und Stickstoff ausgewählt sind, wobei der Substituent oder die Substituenten unter den Halogenatomen, der Nitrogruppe, der Gruppe $CF_3$, Arylgruppen und Heteroarylgruppen ausgewählt sind,
$R^2$ ein Wasserstoffatom oder ein Halogenatom,
$R^3$ ein Wasserstoffatom oder eine Alkylgruppe, Cycloalkylgruppe, Aralkylgruppe oder Arylgruppe, und
$R^4$ eine $C_{1-8}$-Alkylgruppe,

**dadurch gekennzeichnet, dass** die beiden ersten Syntheseschritte des Anspruchs 1 durchgeführt werden.

**17.** Amidine der allgemeinen Formel:

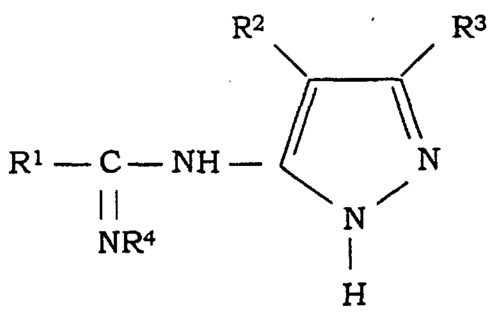

(V),

worin bedeuten:

$R^1$ eine unsubstituierte oder substituierte Aryl- oder Heteroarylgruppe, wobei der Substituent oder die Substituenten unter den Halogenatomen, der Nitrogruppe, der Gruppe $CF_3$, Arylgruppen und Heteroarylgruppen ausgewählt sind,
$R^2$ ein Wasserstoffatom oder ein Halogenatom,
$R^3$ ein Wasserstoffatom oder eine Alkylgruppe, Cycloalkylgruppe, Aralkylgruppe oder Arylgruppe, und
$R^4$ eine $C_{1-8}$-Alkylgruppe,

mit der Maßgabe, dass die Arylgruppe eine Phenylgruppe oder Naphthylgruppe ist und die Heteroarylgruppe Furan, Thiophen, Pyridin oder Pyrimidin bedeutet.

**18.** Amidine nach Anspruch 17, **dadurch gekennzeichnet, dass** der Substituent $R^1$ oder die Substituenten $R^1$ unter den Halogenatomen, der Nitrogruppe und der Gruppe $CF_3$ ausgewählt sind.

**19.** Amidine nach Anspruch 17 oder 18, **dadurch gekennzeichnet, dass** $R^1$ eine substituierte oder unsubstituierte Arylgruppe ist.

**20.** Amidine nach Anspruch 17, 18 oder 19, **dadurch gekennzeichnet, dass** $R^3$ ein Wasserstoffatom oder eine $C_{1-4}$-Alkylgruppe bedeutet.

**21.** Amidine nach Anspruch 17, **dadurch gekennzeichnet, dass** $R^1$ 4-Nitrophenyl, $R^2$ Wasserstoff und $R^3$ *t*-Butyl bedeutet.

## Claims

**1.** Process for preparing carboxamide-oximes represented by the general formula

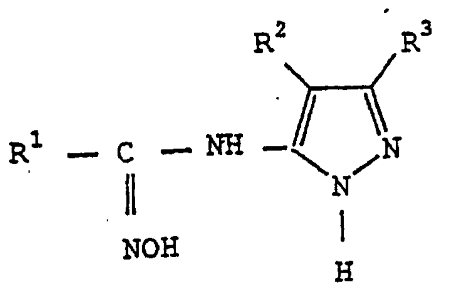

(I)

in which

$R^1$ represents an aryl or heteroaryl group comprising one or more hetero atoms chosen from oxygen, sulfur and nitrogen, which may be substituted or unsubstituted, the substituent(s) being chosen from the group consisting of halogen atoms, a nitro group, a $CF_3$ group, aryl groups and heteroaryl groups,

$R^2$ represents a hydrogen atom or a halogen atom,

$R^3$ represents a hydrogen atom or an alkyl, cycloalkyl, aralkyl or aryl group,

**characterized by** the following steps:

1) an amide represented by the formula $R^1CONHR^4$ (II) in which $R^1$ has the meaning given above and $R^4$ represents a $C_1$ to $C_8$ alkyl group, is first reacted with a chlorinating agent, optionally in the presence of an inert solvent, to give the corresponding chloroimine, represented by formula (III)

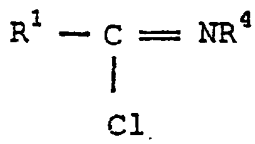

(III)

2) the chloroimine obtained is then reacted with an aminopyrazole represented by formula (IV)

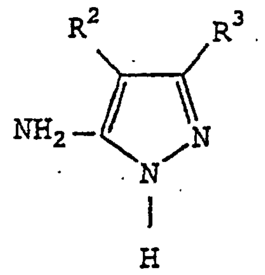

(IV)

in which $R^2$ and $R^3$ have the meanings given above, optionally in the presence of an inert solvent, to give a amidine represented by formula (V)

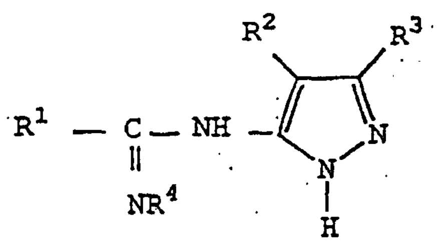

(V)

in which $R^1$, $R^2$, $R^3$ and $R^4$ have the meanings given above,
3) the amidine obtained is reacted with hydroxylamine or a salt thereof, optionally in the presence of an inert solvent.

**2.** Process according to Claim 1, **characterized in that** the substituent(s) of $R^1$ is (are) chosen from the group consisting of halogen atoms, a nitro group and a $CF_3$ group.

**3.** Process according to Claim 1 or 2, **characterized in that** $R^1$ represents a substituted or unsubstituted aryl group.

**4.** Process according to Claim 1, 2 or 3, **characterized in that** $R^3$ represents a hydrogen atom or a $C_1$ to $C_4$ alkyl group.

**5.** Process according to Claim 1, **characterized in that** $R^1$ represents a 4-nitrophenyl group, $R^2$ represents a hydrogen atom and $R^3$ represents a tert-butyl group.

**6.** Process according to any one of the preceding claims, **characterized in that** step 1) is performed with a stoichiometric amount or an excess of a chlorinating agent chosen from the group consisting of thionyl chloride, phosphorus pentachloride, phosphorus oxychloride and phosgene, and a mixture thereof, at a temperature of between about 25°C and the reflux temperature of the solvent.

**7.** Process according to Claim 6, **characterized in that** the chlorinating agent is thionyl chloride.

**8.** Process according to Claim 7, **characterized in that** the reaction is performed in the presence of a catalyst chosen from N,N-dialkyl amides.

**9.** Process according to any one of the preceding claims, **characterized in that** the solvent of step 1) is chosen from the group consisting of the chlorinating agent, chlorinated or non-chlorinated aromatic hydrocarbons and chlorinated or non-chlorinated aliphatic hydrocarbons.

**10.** Process according to any one of the preceding claims, **characterized in that**, in step 2), the aminopyrazole is reacted with the chloroimine, in stoichiometric amount or in excess, at a temperature ranging from -10°C to +30°C.

**11.** Process according to any one of the preceding claims, **characterized in that** step 2) is performed in the presence of a solvent chosen from the group consisting of $C_1$ to $C_8$ aliphatic alcohols, chlorinated or non-chlorinated aromatic hydrocarbons, chlorinated or non-chlorinated aliphatic hydrocarbons, ethers and esters.

**12.** Process according to Claim 11, **characterized in that** the reaction is performed in the presence of an alcohol.

**13.** Process according to any one of the preceding claims, **characterized in that**, in step 3), the hydroxylamine or a salt thereof is reacted, in stoichiometric amount or in excess, at a temperature ranging from 0°C to 60°C.

**14.** Process according to any one of the preceding claims, **characterized in that**, in step 3), hydroxylamine hydrochloride is reacted, in the presence of methanol and a base.

**15.** Process according to any one of the preceding claims, **characterized in that** the chloroimine and the amidine respectively obtained in steps 1) and 2) are not isolated.

**16.** Process for preparing amidines represented by the general formula (V)

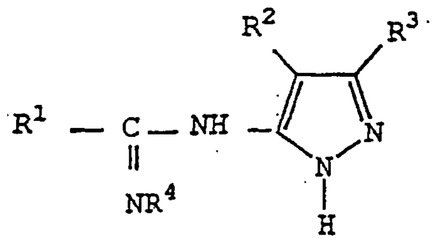

(V)

in which

$R^1$ represents an aryl or heteroaryl group comprising one or more hetero atoms chosen from oxygen, sulfur and nitrogen, which may be substituted or unsubstituted, the substituent(s) being chosen from the group consisting of halogen atoms, a nitro group, a $CF_3$ group, aryl groups and heteroaryl groups,

$R^2$ represents a hydrogen atom or a halogen atom,

$R^3$ represents a hydrogen atom or an alkyl, cycloalkyl, aralkyl or aryl group, and

$R^4$ represents a $C_1$ to $C_8$ alkyl group,

**characterized in that** the first two synthetic steps of Claim 1 are performed.

**17.** Amidines represented by the general formula

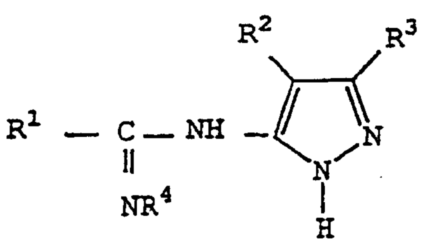

(V)

in which

$R^1$ represents an aryl or heteroaryl group, which may be substituted or unsubstituted, the substituent(s) being chosen from the group consisting of halogen atoms, a nitro group, a $CF_3$ group, aryl groups and heteroaryl groups,

$R^2$ represents a hydrogen atom or a halogen atom,

$R^3$ represents a hydrogen atom or an alkyl, cycloalkyl, aralkyl or aryl group, and

$R^4$ represents a $C_1$ to $C_8$ alkyl group,

it being understood that the aryl group represents a phenyl or naphthyl group and the heteroaryl group represents

furan, thiophene, pyridine or pyrimidine.

**18.** Amidines according to Claim 17, **characterized in that** the substituent(s) of $R^1$ is (are) chosen from the group consisting of halogen atoms, a nitro group and a $CF_3$ group.

**19.** Amidines according to Claim 17 or 18, **characterized in that** $R^1$ represents a substituted or unsubstituted aryl group.

**20.** Amidines according to Claim 17, 18 or 19, **characterized in that** $R^3$ represents a hydrogen atom or a $C_1$ to $C_4$ alkyl group.

**21.** Amidines according to Claim 17, **characterized in that** $R^1$ represents a 4-nitrophenyl group, $R^2$ represents a hydrogen atom and $R^3$ represents a tert-butyl group.